# EUROPEAN PATENT APPLICATION

(11) **EP 3 900 701 A1**
(43) Date of publication of application: **27.10.2021**
(21) Application number: 20170951.6
(22) Date of filing: 22.04.2020
(51) Int. Cl.: A61K 9/00, A61K 9/48

(54) **COMPOSITION OF POMALIDOMIDE FOR ORAL DOSAGE FORM**

(71) Applicant: Lotus Pharmaceutical Co., Ltd., Taipei City 110 (TW)
(72) Inventor: Fang, He Yen, Nantou City, Nantou County 54066 (TW); Gattani, Yogesh Sevaramji, Nantou City, Nantou County 54066 (TW); Gupta, Vijender, Nantou City, Nantou County 54066 (TW); Chawla, Manish, Nantou City, Nantou County 54066 (TW)
(74) Representative: Hartvichova, Katerina

(57) **Abstract**

The present invention relates to a composition for an oral dosage form of pomalidomide which comprises:
- pomalidomide or a pharmaceutically acceptable salt or solvate thereof;
- at least one filler which is isomalt;
- at least one binder which is pregelatinized starch;
- at least one lubricant which is sodium stearyl fumarate.

The composition of the present invention has an improved final dissolution amount in neutral to medium acidic media and excellent stability.

## Description

### Field of Art

The present invention relates to an oral dosage form of pomalidomide with a higher availability of the active substance.

### Background Art

Pomalidomide, chemically named 4-amino-2-(2,6-dioxopiperidine3-yl)isoindole-1,3-dione, is an anti-antiogenic and immunomodulatory drug. Pomalidomide inhibits, inter alia, LPS induced monocyte TNFα, IL-1β, IL-12, IL-6, MIP-1, MCP-1, GM-CSF, G-CSF, COX-2 production, and co-stimulates the activation of T-cells. This combination of properties makes pomalidomide suitable for treatment of cancer, disorders associated with angiogenesis, pain including, but not limited to, Complex Regional Pain Syndrome ("CRPS"), Macular Degeneration ("MD") and related syndromes, skin diseases, pulmonary disorders, asbestos-related disorders, parasitic diseases, immunodeficiency disorders, CNS disorders, CNS injury, atherosclerosis and related disorders, dysfunctional sleep and related disorders, hemoglobinopathy and related disorders (e.g., anemia), TNFα related disorders, and other various diseases and disorders, as described in detail in, e.g., WO 2010/135396. Pomalidomide was approved in 2013 in the US and EU for treatment of relapsed and refractory multiple myeloma.

Active pharmaceutical ingredients are commonly administered in the form of pharmaceutical formulations with excipients, which are called dosage forms. The excipients of the dosage forms contribute to the quality of the dosage form by improving, e.g., stabilization, preservation, flavoring, solubilization of the active ingredient. Further considerations include ease of manufacture, ease of administration, patient compliance etc. Each active pharmaceutical ingredient requires tailoring the dosage form to its unique properties, and to allow full use of the pharmaceutical properties and activities of the active ingredient.

In EU, pomalidomide is marketed by Celgene under the name Imnovid. Imnovid is an oral formulation in the form of hard gelatin capsules filled with a composition of the API with mannitol, pregelatinised starch and sodium stearyl fumarate.

In the pharmaceutical field, there is a continuous need to produce dosage forms with suitable physical, pharmacokinetic and pharmaceutical properties, in order to adapt the formulations for the needs of any patients.

### Disclosure of the Invention

The present invention provides a composition for an oral dosage form of pomalidomide which comprises or consists of:
- pomalidomide or a pharmaceutically acceptable salt or solvate thereof;
- at least one filler which is isomalt;
- at least one binder which is pregelatinized starch;
- at least one lubricant which is sodium stearyl fumarate.

Pharmaceutically acceptable salts of pomalidomide are salts with acids or bases. Suitable acids include maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic, pamoic, hydrochloric, hydrobromic, hydroiodic, sulfuric, phosphoric, nitric acids, and amino acids. Bases include pharmaceutically acceptable cations such as alkali metal or alkaline earth metal cations, particularly, calcium, magnesium, sodium, lithium, potassium cations, zinc cations, iron cations, ammonium cations.

Solvates of pomalidomide or of a salt of pomalidomide further include a stoichiometric or non-stoichiometric amount of a solvent. The solvent may be water, then the solvate can also be called hydrate.

Pomalidomide or the salt or solvate thereof is preferably present in the composition for unit dose in an amount corresponding to 1 to 10 mg of pomalidomide in the composition for unit dose, preferably 1-5 mg of pomalidomide in the composition for unit dose, more preferably about 1 or about 2 or about 3 or about 4 or about 5 mg of pomalidomide in the composition for unit dose.

The amount of isomalt in the composition for unit dose is preferably in the range of 30 to 50 wt. %, more preferably 35 to 45 wt. %, even more preferably 40 to 45 wt. %.

The amount of pregelatinized starch in the composition for unit dose is preferably in the range of 48 to 68 wt. %, more preferably 53 to 63 wt. %, even more preferably 53 to 58 wt. %.

The amount of sodium stearyl fumarate in the composition for unit dose is preferably 0.1 to 1 wt. %, even more preferably 0.2 to 0.6 wt. %.

In some embodiments, the composition for unit dose contains or consists of:
- 1 to 5 mg of pomalidomide or a pharmaceutically acceptable salt or solvate thereof;
- 30 to 50 wt. % of isomalt;
- 48 to 68 wt. % of pregelatinized starch;
- 0.1 to 1 wt. % of sodium stearyl fumarate;
- optionally further excipients.

In some embodiments, the composition for unit dose contains or consists of:
- 1 to 5 mg of pomalidomide or a pharmaceutically acceptable salt or solvate thereof;
- 40 to 45 wt. % of isomalt;
- 53 to 58 wt. % of pregelatinized starch;
- 0.1 to 1 wt. % of sodium stearyl fumarate,
- optionally further excipients.

In some embodiments, the composition for unit dose contains or consists of:
- 1 to 5 mg of pomalidomide or a pharmaceutically acceptable salt or solvate thereof;
- 35 to 45 wt. % of isomalt;
- 53 to 63 wt. % of pregelatinized starch;
- 0.1 to 1 wt. % of sodium stearyl fumarate;
- optionally further excipients.

The total weight of the composition for unit dose comprising pomalidomide or the salt or solvate thereof, isomalt, pregelatinized starch, sodium stearyl fumarate, and optionally further excipients, can be, for example, in the range of 100 to 1000 mg, preferably 100 to 500 mg, more preferably 200 to 300 mg, even more preferably 230 to 250 mg, such as 240 mg.

In a particular embodiment, the composition for unit dose has the following composition:
- 1 to 5 mg of pomalidomide which corresponds to 0.4 to 2.1 wt. %;
- 41 to 43 wt. % of isomalt;
- 55 to 57 wt. % of pregelatinized starch;
- 0.2 to 0.6 wt. % of sodium stearyl fumarate.

In a particular embodiment, the composition for unit dose has the following composition:
- about 1.67 wt. % of pomalidomide;
- about 41.83 wt. % of isomalt;
- about 56 wt. % of pregelatinized starch;
- about 0.50 wt. % of sodium stearyl fumarate.

In one particular embodiment, the composition for unit dose has the following composition:
- about 1.67 wt. % of pomalidomide;
- about 42.08 wt. % of isomalt;
- about 56 wt. % of pregelatinized starch;
- about 0.25 wt. % of sodium stearyl fumarate.

In a particular embodiment, the composition for unit dose, said composition having a total weight of 240 mg, has the following composition:
- 4 mg of pomalidomide which corresponds to about 1.67 wt. %;
- about 41.83 wt. % of isomalt;
- about 56 wt. % of pregelatinized starch;
- about 0.50 wt. % of sodium stearyl fumarate.

In another particular embodiment, the composition for unit dose, said composition having a total weight of 240 mg, has the following composition:
- 4 mg of pomalidomide which corresponds to about 1.67 wt. %;
- about 42.08 wt. % of isomalt;
- about 56 wt. % of pregelatinized starch;
- about 0.25 wt. % of sodium stearyl fumarate.

The oral dosage form is preferably a solid oral dosage form selected from a hard capsule, a soft capsule, a caplet, a sachet, a tablet, and a coated tablet. The capsules may be, for example, gelatin capsules. In case of capsules, the amounts and weight percents listed above for the "composition for unit dose" do not include the capsule shell. In case of coated tablets, the amounts and weight percents listed above for the "composition for unit dose" do not include the coating.

The oral dosage form of the composition of the present invention is preferably packaged and stored in conditions which decrease or prevent exposure to water. Suitable packaging may include, for example, hermetically sealed foils, plastics, blister packs, strip packs, closed vials and bottles with dessicator, unit dose containers.

The term "about" contemplates that an amount or weight percent within 20 % or 15 % or 10 % or 5 % of the specified amount of weight percent is encompassed.

The composition of the present invention, or the oral dosage form comprising such composition is particularly suitable for use in the treatment of cancer, disorders associated with angiogenesis, pain including, but not limited to, Complex Regional Pain Syndrome ("CRPS"), Macular Degeneration ("MD") and related syndromes, skin diseases, pulmonary disorders, asbestos-related disorders, parasitic diseases, immunodeficiency disorders, CNS disorders, CNS injury, atherosclerosis and related disorders, dysfunctional sleep and related disorders, hemoglobinopathy and related disorders (e.g., anemia), TNFα related disorders, and other various diseases and disorders, as described in detail in, e.g., WO 2010/135396.

In particular, composition of the present invention, or the oral dosage form comprising such composition is suitable for use in for treatment of relapsed and refractory multiple myeloma.

Isomalt is a sugar alcohol used, inter alia, in food and pharmaceutical industry. It has little to no impact on blood sugar levels and is thus suitable also for patients suffering from diabetes. Isomalt is a mixture (usually equimolar mixture) of two mutually diastereomeric disaccharides: 6-0-α-D-glucopyranosyl-D-sorbitol and 1-O-α-D-glucopyranosyl-D-mannitol. Isomalt is non-hygroscopic and does not significantly absorb additional water until 85 % relative humitidy at 25 °C. It has a very good thermal and chemical stability, and exhibits considerable resistance to acids and microbial influences.

Compared to the commercially available formulation Imnovid of pomalidomide which contains mannitol, pregelatinized starch and sodium stearyl fumarate, the composition of the present invention (containing isomalt instead of mannitol) allows to achieve a more complete dissolution of the active ingredient after 60 minutes (final dissolution rate) in medium acidic to neutral media, while maintaining bioequivalence and showing a similar dissolution profile with the reference product. Furthermore, the dosage form has a high stability with almost no formation of impurities above a detectable level - only L-glutamine appeared upon storage, in very low amounts. The stability of the novel product is higher than that of the reference product (RLD).

Any % mentioned in this text is wt.%, unless specified otherwise.

### Brief description of Drawings

Figure 1: Dissolution profile in discriminatory media according to Example 3
Figure 2: Dissolution profile in release media according to Example 4
Figure 3: Dissolution profile in pH 4.5 according to Example 5
Figure 4: Dissolution profile in pH 6.8 according to Example 6

### Examples of carrying out the Invention

### Example 1: Preparation of the composition

Composition of the unit dose composition is as follows:

| | | Example 1A | | Example 1B | |
|---|---|---|---|---|---|
| Component | Function | mg/ capsule | wt. % | mg/ capsule | wt. % |
| Pomalidomide | API | 4.0 | 1.67 | 4.0 | 1.67 |
| Pregelatinized starch | binder/filler | 134.4 | 56.00 | 134.4 | 56.00 |
| Isomalt 801 | filler | 10.0 | 4.17 | 10.0 | 4.17 |
| Isomalt 721 | filler | 91.0 | 37.91 | 90.4 | 37.66 |
| Sodium stearyl fumarate | lubricant | 0.6 | 0.25 | 1.2 | 0.50 |
| Fill weight (mg) | | 240.0 | 100 | 240.0 | 100 |
| Hard gelatine capsule shell size 2 | | 1 pc | | 1 pc | |

Pomalidomide was blended with isomalt 801 and one third of the amount of pregelatinized starch in an octagonal blenter at 29 rpm for 6 minutes. The mixture was sieved through a 30 mesh sieve, blended with one third of the amount of pregelatinized starch and one half of the amount of isomalt 721 at 26 rpm for 10 minutes, and sieved through a 30 mesh sieve. The last third of the amount of pregelatinized starch and the remaining half of the amount of isomalt 721 were added, and the mixture was blended at 26 rpm for 30 minutes, sieved through a 60 mesh sieve, and blended with sodium stearyl fumarate at 26 rpm for 5 minutes. The final blend was filled into a hard gelatine capsule shell size 2.

### Example 2: Comparative example - reference listed drug composition (RLD, Imnovid)

| Component | Function | mg/ capsule | wt. % |
|---|---|---|---|
| Pomalidomide | API | 4.0 | 1.67 |
| Pregelatinized starch | binder/filler | 134.4 | 56.00 |
| Mannitol | filler | 101.0 | 42.08 |
| Sodium stearyl fumarate | lubricant | 0.6 | 0.25 |
| Fill weight (mg) | | 240.0 | 100 |
| Hard gelatine capsule shell size 2 | | 1 pc | |

### Example 3: Dissolution profile in discriminatory media (USP-IV)

Dissolution media preparation:
0.01N Hydrochloric acid: Transfer 8.5 mL HCl in sufficient amount of purified water. Dilute to 10L with purified water and mix well.
pH 5.0 Buffer + Sodium Chloride: Weigh 118 g NaCl, 40 g NaOH, 87 mL Acetic acid to 10L of Purified water, mix well. Adjust to pH 5.0±0.05 with NaOH solution.
pH 6.5 Buffer + Sodium Chloride: Weigh 61.9 g NaCl, 39.5 g NaH₂PO₄ to 10L of Purified water, mix well. Adjust to pH 6.5±0.05 with NaOH solution.

Conditions:
1. Temp.: 37°C ± 0.5°C
2. Apparatus:- USP IV
3. Flow rate: 8.0 mL/min
4. Sampling volume: lOmL
5. Medium / Sampling time point
0.01 N Hydrochloric acid: At 10, 20, 30 minutes
pH 5.0 buffer: at 40 & 60 minutes (Continuous change medium)
pH 6.5 buffer : At 90 & 120 minutes (Continuous change medium)

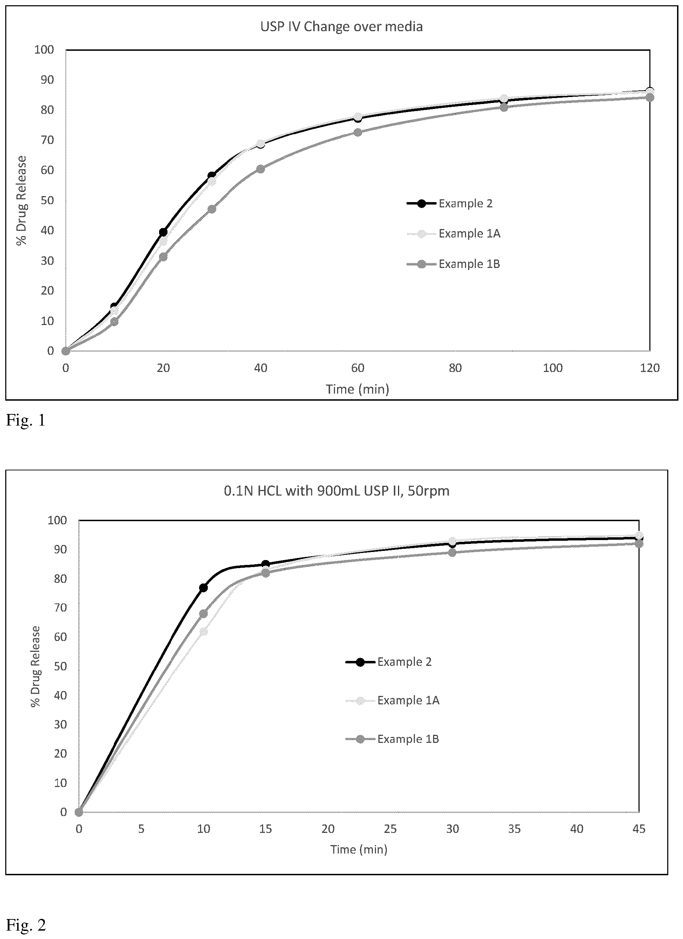

The obtained dissolution profile for both the composition of the present invention and the comparative composition (RLD) is summarized in the table herein below, as well as in Figure 1.

| | | RLD (Example 2) n = 2 | | Composition of Example 1 A n = 3 | | Composition of Example 1 B n = 2 | |
|---|---|---|---|---|---|---|---|
| | Time(min) | Mean | RSD | Mean | RSD | Mean | RSD |
| 0.01N HCl | **0** | 0 | 0.0 | 0 | 0.0 | 0 | 0 |
| | **10** | 15 | 6.7 | 13 | 12.0 | 10 | 24.9 |
| | **20** | 40 | 1.5 | 37 | 5.5 | 31 | 6.8 |
| | **30** | 58 | 1.5 | 56 | 4.0 | 47 | 7.3 |
| pH 5.0 Buffer | **40** | 69 | 1.8 | 69 | 4.2 | 60 | 8.1 |
| | **60** | 77 | 2.5 | 78 | 5.0 | 73 | 6.3 |
| pH 6.5 Buffer | **90** | 83 | 3.2 | 84 | 5.8 | 81 | 3.1 |
| | **120** | 86 | 3.2 | 86 | 6.1 | 84 | 3.2 |

### Example 4: Dissolution profile in release media (0.1N HCl with 900 ml USP II)

Dissolution medium preparation:
0.1N Hydrochloric acid: Transfer 85 mL Hydrochloride acid and dilute to 10L with purified water and degas

Conditions:
1. Medium: - 0.1N Hydrochloride acid
2. Apparatus: - II (Paddle) with Sinker
3. Temp.: 37°C ± 0.5°C
4. Rotation speed: - 50rpm
5. Volume: 900mL
6. Sampling volume: lOmL
7. Sampling time point: - 10, 15, 20, 30, 45, 60min

The obtained dissolution profile for both the composition of the present invention and the comparative composition (RLD) is summarized in the table herein below, as well as in Figure 2.

| | | RLD (Example 2) n = 6 | | Composition of Example 1 A n = 6 | | Composition of Example 1 B n = 3 | |
|---|---|---|---|---|---|---|---|
| | Time(min) | Mean | RSD | Mean | RSD | Mean | RSD |
| 0.1N Hydrochloride acid | 0 | 0 | 0 | 0 | 0 | 0 | 0 |
| | 10 | 77 | 4.4 | 62 | 16.0 | 68 | 13.6 |
| | 15 | 85 | 2.6 | 83 | 5.3 | 82 | 4.4 |
| | 30 | 92 | 2.5 | 93 | 2.0 | 89 | 1.6 |
| | 45 | 94 | 2.6 | 95 | 1.7 | 92 | 1.7 |

### Example 5: Dissolution profile in pH 4.5 (acetate buffer, with 900 ml USP II)

Dissolution medium preparation:
Weigh 2.99 g of Sodium acetate into a 1000 mL volumetric flask, add 14 mL of 2N Acetic acid, and add 750 mL of Purified water to dissolve. Dilute to volume with purified water, mix well. Adjust to pH 4.5±0.05 with 2N Acetic acid or 0.2M NaOH

Condition:-
1. Medium: pH 4.5 (acetate buffer)
2. Apparatus: II (Paddle) with Sinker
3. Temp.: 37°C ± 0.5°C
4. Rotation speed: - 50rpm
5. Volume: 900mL
6. Sampling volume: lOmL
7. Sampling time point: - 10, 15, 20, 30, 45, 60min

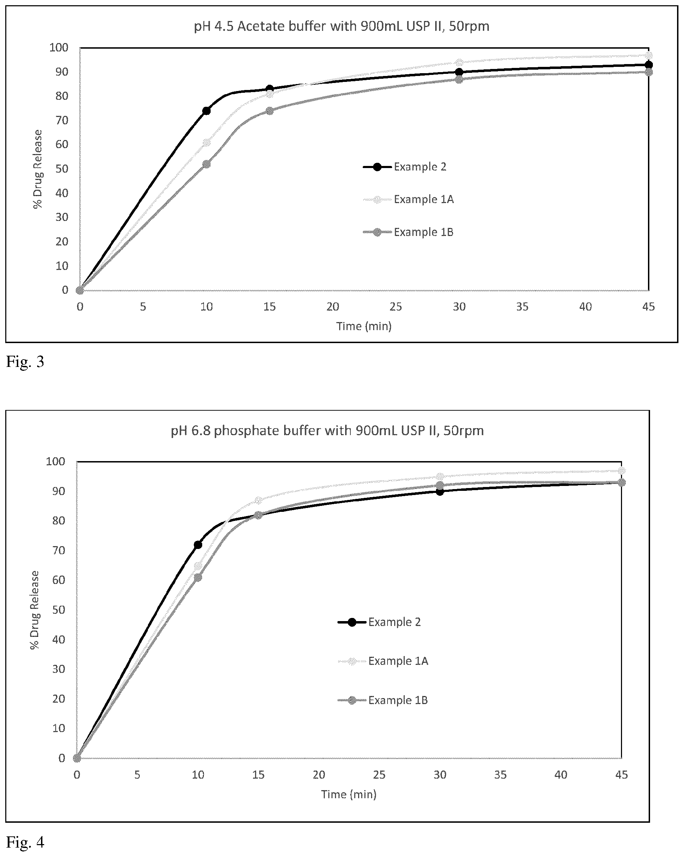

The obtained dissolution profile for both the composition of the present invention and the comparative composition (RLD) is summarized in the table herein below, as well as in Figure 3.

| | | RLD (Example 2) n = 3 | | Composition of Example 1A n = 3 | | Composition of Example 1B n = 3 | |
|---|---|---|---|---|---|---|---|
| | Time(min) | Mean | RSD | Mean | RSD | Mean | RSD |
| pH 4.5 Acetate buffer | 0 | 0 | 0 | 0 | 0 | 0 | 0 |
| | 10 | 74 | 4.8 | 61 | 33.1 | 52 | 6.8 |
| | 15 | 83 | 3.1 | 81 | 13.7 | 74 | 2.1 |
| | 30 | 90 | 2.5 | 94 | 4.4 | 87 | 1.3 |
| | 45 | 93 | 2.2 | 97 | 2.8 | 90 | 1.1 |

### Example 6: Dissolution profile in pH 6.8 (phosphate buffer, with 900 ml USP II)

Weigh 6.9 g of Monobasic potassium phosphate in sufficient amount of purified water. Add 112 ml of 0.2M NaOH, dilute to 1000 mL with purified water and mix well. Adjust to pH 6.8±0.05 with Phosphoric acid or 0.2M NaOH

Conditions:
1. Medium: - pH 6.8 Phosphate buffer
2. Apparatus: - II (Paddle) with Sinker
3. Temp.: 37°C ± 0.5°C
4. Rotation speed: - 50rpm
5. Volume: 900mL
6. Sampling volume: lOmL
7. Sampling time point: - 10, 15, 20, 30, 45, 60min

The obtained dissolution profile for both the composition of the present invention and the comparative composition (RLD) is summarized in the table herein below, as well as in Figure 4.

| | | RLD (Example 2) n = 3 | | Composition of Example 1A n = 3 | | Composition of Example 1B n = 3 | |
|---|---|---|---|---|---|---|---|
| | Time(min) | Mean | RSD | Mean | RSD | Mean | RSD |
| pH 6.8 Phosphate buffer | 0 | 0 | 0 | 0 | 0 | 0 | 0 |
| | 10 | 72 | 0.5 | 65 | 9.0 | 61 | 9.0 |
| | 15 | 82 | 1.1 | 87 | 3.1 | 82 | 2.5 |
| | 30 | 90 | 2.4 | 95 | 2.2 | 92 | 0.6 |
| | 45 | 93 | 2.9 | 97 | 1.8 | 93 | 1.0 |

### Example 7: Stability measurement

The composition prepared according to Example 1 and filled into hard capsule was used for the stability measurement. The samples were stored at 40±2 °C and 75%±5% relative humidity.

Water Content: Transfer about 30 to 40 mL of methanol to titration vessel and titrate with the Karl Fischer reagent to the electrometric to consume any moisture that may be present. Trnasfer and weigh approximately 250 mg of sample powder and quickly transfer into titration vessel, then titrate with the Karl Fischer reagent to the electrometric point.

Impurities Stock Solutions: Accurately weigh 1.0 mg of each measured imputiry and transfer into a 100 M1 volumetric flask. Add about 70 mL of diluent and sonicate for 10 minutes to dissolve complete. Dilute up to mark with diluent and mix well.

API Stock Solution: Accurately weigh 12.5 mg of pomalidomide standard into a 50 mL volumetric flask. Add about 40 mL of diluent and sonicate for 15 minutes at ambient temperature with intermittent shaking for every three minutes. Dilute up to the mark with diluent and mix well.

Sample Solution: Randomly select minimum 20 capsules for each strength and remove the content to make homogeneous mixture. Accerately weigh about 750 mg of sample powder and transfer into 25 mL volumetric flask. Add 20 mL of diluent and sonicate for 15 minutes with intermittent shaking for every three minutes at ambient temperature. Make up to volume with diluent and mix well. Filter the solution through 0.45 micrometer Nylon filter and discard first 3 mL of the filtrate.

The content of impurities was measured by HPLC with gradient of mobile phases A and B. Mobile phase A: Transfer 1.0 mL of ortho-phosphoric acid (85%) into 1000 mL of purified water. Adjust with 85% phosphoric acid to pH 1.90±0.05. Filter the solution through 0.45 micrometer Nylon memberane filter.

Mobile phase B: Mix acetonitrile and Mobile phase A in the ratio of 60:40 (v/v). Diluent: Prepare a mixture of acetonitrile and purified water in the ratio of 80:20 (v/v). Chromatographic conditions:

| S. No. | Particulars | Details |
|---|---|---|
| 1 | Column | Kromasil 100-5-C18, 4.6x250 mm, 5 µm or equivalent |
| 2 | Column temperature | 40 °C |
| 3 | Wavelength | UV at 220 nm |
| 4 | Flow rate | 1.0 mL/minute |
| 5 | Autosampler temperature | 10 °C |
| 6 | Injection volume | 5 µL |
| 7 | Run time | 40 minutes, make adjustment if necessary |
| 8 | Pump mode | Gradient |
| 9 | Washing solvent | Acetonitrile : Water (50:50 % v/v) |

Gradient Programme:

| Flow rate (mL/minute) | Time (minutes) | Mobile Phase A (%) | Mobile Phase B (%) |
|---|---|---|---|
| 1.0 | 0.0 | 90 | 10 |
| 1.0 | 2 | 80 | 20 |
| 1.0 | 20 | 30 | 70 |
| 1.0 | 25 | 20 | 80 |
| 1.0 | 32 | 20 | 80 |
| 1.0 | 32.1 | 90 | 10 |
| 1.0 | 40 | 90 | 10 |

The results of the stability measurements are presented herein below. N.D. = not detected.

| Specifications | Time (months) | | | |
|---|---|---|---|---|
| | 0 | 1 | 2 | 3 |
| API (90.0% to 110%) | 103.4% | 103.3% | 102.3% | 103.0% |
| L-glutamine (Not more than 0.5%) | N.D. | 0.1% | 0.071% | 0.062% |
| 3-aminophthalic acid (Not more than 0.5%) | N.D. | N.D. | N.D. | N.D. |
| 3-nitrophthalic acid (not more than 0.5%) | N.D. | N.D. | N.D. | N.D. |
| hydroxylamine impurity (not more than 0.5%) | N.D. | N.D. | N.D. | N.D. |
| nitro impurity (not more than 0.5%) | N.D. | N.D. | N.D. | N.D. |
| dimethyl glyoxime (not more than 0.5%) | N.D. | N.D. | N.D. | N.D. |
| benzyldione impurity (not more than 0.5%) | N.D. | N.D. | N.D. | N.D. |
| any unspecified impurity (not more than 0.5%) | 0.036% | 0.038% | 0.017% | 0.023% |
| water content | 5.9% | 5.8% | 5.7% | 5.3% |

### Example 8: Bioequivalence study

Study Title: An open label, randomized, balanced, two-treatment, two-period, two-sequence, single-dose, crossover, pilot, oral bioequivalence study of Pomalidomide 4 mg hard capsules from Lotus Pharmaceutical Co., Ltd., Taiwan and Imnovid (Pomalidomide) 4 mg hard capsules of Celgene Europe, in healthy, adult, human subjects under fasting conditions.

Study Number: NCS-668-19-CS

Primary objective: To characterize the rate and extent of Pomalidomide absorption after oral administration and to assess the bioequivalence of the sponsor's test product Pomalidomide 4 mg hard capsules and the reference product Imnovid (Pomalidomide) 4 mg hard capsules, in healthy, adult, human subjects under fasting conditions.

Secondary objective: To assess the safety and tolerability of both the formulations on the basis of clinical and laboratory examination, documentation of the Adverse Events (AEs) and/or Adverse Drug Reactions (ADRs).

Study Design: An open label, randomized, balanced, two-treatment, two-period, two-sequence, single-dose, crossover, pilot, oral bioequivalence study with a washout period of at least 07 days in healthy, adult, human subjects under fasting conditions.

Sample Size: 14 healthy, adult, human subjects.

Investigational Products:
**Test product:** Pomalidomide 4 mg hard capsules
   Manufactured by: Lotus Pharmaceutical Co., Ltd., Taiwan
**Reference product:** Imnovid (Pomalidomide) 4 mg hard capsules
   Manufactured by: Celgene Distribution B.V., Netherlands
   Marketing Authorization Holder: Celgene Europe B.V., Netherlands
**Drug administration:** In each period, following an overnight fast of at least 10 hours, subjects were received a single oral dose of either the test product or reference product while in a sitting posture with about 240 ± 2 mL of water at ambient temperature. Drug administration was done as per the randomization schedule generated by RStudio version 1.2 or higher.
   Note: Drug administration was done under yellow monochromatic light.
**Blood Sampling:** In each period, 24 blood samples were collected from each subject. The predose (0.000 hour) blood sample (4 mL) were collected within 1 hour prior to dosing.
   The post-dose blood samples (4 mL) were collected at 0.333, 0.667, 1.000, 1.250, 1.500, 1.750, 2.000, 2.250, 2.500, 2.750, 3.000, 3.500, 4.000, 5.000, 6.000, 7.000, 8.000, 10.000, 12.000, 16.000, 24.000, 36.000 and 48.000 hours.
**Washout period:** at least 07 days.
**Bio-Analytical Methods:** The plasma concentrations of Pomalidomide were quantified by a validated LCMS/MS analytical method in accordance with Bioanalytical SOPs of Norwich Clinical Services.
   Note: The samples were processed and analyzed under yellow monochromatic light.

| Dependent | Geometric LSM Reference (R) | Geometric LSM Test (T) | Ratio (%) | CI_90_Lower (%) | CI_90_Upper (%) | ISCV (%) | Power (%) |
|---|---|---|---|---|---|---|---|
| Cmax (ng/ml) | 68.903 | 76.295 | 110.73 | 103.41 | 118.57 | 10.18 | 99.92 |
| AUCT (hr^{∗}ng/ml) | 624.140 | 660.603 | 105.84 | 100.96 | 110.96 | 7.02 | 100.00 |

**Result Conclusion:** The ratio and 90% confidence interval of the test/reference (T/R) ratio (difference in least square means) from the ANOVA of the log transformed data for the pharmacokinetic parameters AUCO-t and Cmax for Pomalidomide estimated and found within the acceptable range of 80.00% to 125.00%, hence the Test product is bioequivalent with that of Reference product.

## Claims

1. A composition for an oral dosage form of pomalidomide which comprises:
- pomalidomide or a pharmaceutically acceptable salt or solvate thereof;
- at least one filler which is isomalt;
- at least one binder which is pregelatinized starch;
- at least one lubricant which is sodium stearyl fumarate.

2. The composition according to claim 1, wherein pomalidomide or the salt or solvate thereof is present in an amount corresponding to 1 to 10 mg of pomalidomide in the composition for unit dose, preferably 1-5 mg of pomalidomide in the composition for unit dose, more preferably about 1 or about 2 or about 3 or about 4 or about 5 mg of pomalidomide in the composition for unit dose.

3. The composition according to any one of the preceding claims, wherein the amount of isomalt in the composition is in the range of 30 to 50 wt. %, more preferably 35 to 45 wt. %, even more preferably 40 to 45 wt. %.

4. The composition according to any one of the preceding claims, wherein the amount of pregelatinized starch in the composition is in the range of 48 to 68 wt. %, more preferably 53 to 63 wt. %, even more preferably 53 to 58 wt. %.

5. The composition according to any one of the preceding claims, wherein the amount of sodium stearyl fumarate in the composition is 0.1 to 1 wt. %, preferably 0.2 to 0.6 wt. %, even more preferably 0.2 to 0.3 wt. %.

6. The composition according to claim 1, wherein the composition contains per unit dose:
- 1 to 5 mg of pomalidomide or a pharmaceutically acceptable salt or solvate thereof;
- 30 to 50 wt. % of isomalt;
- 48 to 68 wt. % of pregelatinized starch;
- 0.1 to 1 wt. % of sodium stearyl fumarate;
- optionally further excipients.

7. The composition according to claim 1, wherein the composition contains per unit dose:
- 1 to 5 mg of pomalidomide or a pharmaceutically acceptable salt or solvate thereof;
- 40 to 45 wt. % of isomalt;
- 53 to 58 wt. % of pregelatinized starch;
- 0.1 to 1 wt. % of sodium stearyl fumarate,
- optionally further excipients.

8. The composition according to claim 1, wherein the composition contains per unit dose:
- 1 to 5 mg of pomalidomide or a pharmaceutically acceptable salt or solvate thereof;
- 35 to 45 wt. % of isomalt;
- 53 to 63 wt. % of pregelatinized starch;
- 0.1 to 1 wt. % of sodium stearyl fumarate;
- optionally further excipients.

9. The composition according to claim 1, wherein the composition contains per unit dose:
- 1 to 5 mg of pomalidomide which corresponds to 0.4 to 2.1 wt. %;
- 41 to 43 wt. % of isomalt;
- 55 to 57 wt. % of pregelatinized starch;
- 0.2 to 0.6 wt. % of sodium stearyl fumarate.

10. The composition according to claim 1, wherein the composition contains per unit dose:
- about 1.67 wt. % of pomalidomide;
- about 41.83 wt. % of isomalt;
- about 56 wt. % of pregelatinized starch;
- about 0.50 wt. % of sodium stearyl fumarate.

11. The composition according to claim 1, wherein the composition contains per unit dose:
- about 1.67 wt. % of pomalidomide;
- about 42.08 wt. % of isomalt;
- about 56 wt. % of pregelatinized starch;
- about 0.25 wt. % of sodium stearyl fumarate.

12. An oral dosage form comprising the composition according to any one of the preceding claims, wherein the oral dosage form is a solid oral dosage form selected from a hard capsule, a soft capsule, a caplet, a sachet, a tablet, and a coated tablet.

13. The composition of any one of claims 1 to 11, or the oral dosage form according to claim 12, for use in a method of treatment of cancer, disorders associated with angiogenesis, pain including complex regional pain syndrome, macular degeneration, skin diseases, pulmonary disorders, asbestos-related disorders, parasitic diseases, immunodeficiency disorders, CNS disorders, CNS injury, atherosclerosis, dysfunctional sleep, hemoglobinopathy, anemia, TNFα related disorders.

14. The composition of any one of claims 1 to 11, or the oral dosage form according to claim 12, for use in a method of treatment of relapsed and refractory multiple myeloma.
